# EUROPEAN PATENT SPECIFICATION

(11) **EP 2 618 819 B1**
(45) Date of publication and mention of the grant of the patent: **01.11.2017**
(21) Application number: 11776879.6
(22) Date of filing: 21.09.2011
(51) Int. Cl.: A61K 9/28, A61K 31/437, A61K 31/44, A61K 9/20, A61K 9/16, A61K 9/00

(54) **PHARMACEUTICAL FORMULATIONS CONTAINING RIFAXIMIN, PROCESSES FOR THEIR OBTAINMENT AND METHOD OF TREATING INTESTINAL DISEASE**
PHARMAZEUTISCHE FORMULIERUNGEN MIT RIFAXIM, VERFAHREN FÜR IHRE GEWINNUNG UND VERFAHREN ZUR BEHANDLUNG VON DARMERKRANKUNGEN
FORMULATIONS PHARMACEUTIQUES CONTENANT DE LA RIFAXIMINE, PROCÉDÉS POUR LEUR OBTENTION ET PROCÉDÉ DE TRAITEMENT DE MALADIES INTESTINALES

(30) Priority: 19.01.2011 IT BO20110012; 22.10.2010 IT BO20100638; 22.09.2010 IT BO20100567
(43) Date of publication of application: 31.07.2013
(73) Proprietor: Alfasigma S.p.A., 20126 Milano (IT)
(72) Inventor: GIUSEPPE CLAUDIO, Viscomi, I-40133 Bologna (IT); PAOLA, Maffei, I-40133 Bologna (IT); GIUSEPPE, Bottoni, I-40133 Bologna (IT); MARIA, Grimaldi, I-40133 Bologna (IT)
(74) Representative: Hiebl, Inge Elisabeth
(86) International application number: PCT/IB2011/054133
(87) International publication number: WO 2012/038898

(56) References cited:
- WO-A1-2008/029208
- WO-A1-2009/008005
- WO-A2-2006/094737
- US-A1- 2005 101 598
- US-A1- 2008 262 012

## Description

### Object of the Invention

The object of the invention concerns gastroresistant tablets containing rifaximin, obtained by means of gastroresistant microgranules characterized in that they inhibit the rifaximin release at pH values between 1.5 and 4.0, and they allow its release at pH values between 5.0 and 7.5, the processes for their obtainment and their use in the treatment and the prevention of diseases directly or indirectly deriving from inflammatory bowel diseases.

### Background of the Disclosure

Among the pathologies of the digestive apparatus those having an infective etiology and/or an inflammatory bowel etiology are frequent. They include bowel infections, irritable bowel syndromes and inflammatory bowel diseases.

The term "bowel infections" comprises, for example, diarrheic syndromes, traveler's diarrhea, diarrheas caused by an altered balance of intestinal microbial flora, enteritis. The term inflammatory bowel diseases also indicates, for example, Crohn's disease and ulcerous recto-colitis. Other bowel pathologies comprise irritable bowel syndromes (IBS) also called "irritable bowel disease" the bacterial overgrowth in the small intestine, also called "small intestinal bacterial overgrowth" (SIBO) and diverticular syndromes.

The etiology of the aforesaid bowel pathologies is likely to be attributed, at least partially, to bacterial pathogenic agents or to intestinal bacterial flora alterations. Hepatic encephalopathy is also treated with rifaximin since the pathology itself and its complications may occur by the ammonia production by the intestinal microbiota.

In the case of inflammatory bowel diseases, the aim of the medical therapy is reducing the inflammatory status by using drugs such as antibiotics, steroids, 5-aminosalicylates (5-ASA), immunosupressants, specific chemotherapics and the so-called biological drugs, such as e.g. infliximab, etanercept, adalimumab, anakirna.

In this scope, antibiotics are widely used with the aim of reducing the bacterial burden in the intestinal lumen and, therefore, the inflammatory status it involves, with the following remission of the symptomatology associated to this pathologic status (diarrhea, bowel pains, meteorism, tenesmus, abscesses, fistulae, etc.).

The used drugs are frequently associated with severe side effects and/or adverse reactions, also leading to therapy suspension. Therefore, there is the need of having new available drugs which are effective in treating such pathologies and that have a suitable safety profile which also allows for their chronic use.

Rifaximin is an antibiotic active against gram-positive and gram-negative bacteria which has been reported to be not absorbed, as described by Descombe J. et al. in Int. J. Clin. Pharmacol. Res., 14 (2), 51-56, (1994) and act only locally in the gastro-intestinal duct. Due to its antibiotic and local activity, rifaximin plays a relevant role in the therapy against infective and inflammatory bowel diseases, both in their acute and in chronic phases.

Rifaximin exists in crystalline and amorphous forms which are described in, for example, U.S. Patent No. 7,045,620 and U.S. Patent Applications having publication nos. US20080262024 and US20090130201. The different forms of rifaximin are associated with different levels of absorption into blood. See, U.S. Patent Application No. US200801332530. Presently, rifaximin is authorized for treating pathologies whose etiology is partly or completely referable to acute and chronic bowel diseases caused by gram-positive and gram-negative bacteria, such as diarrheic syndromes, traveler's diarrhea, diarrheas caused by an altered balance of intestinal microbial flora such as summer diarrheas, traveler's diarrhea, enterocolitis. Moreover, it is used in the pre- and post-surgical prophylaxis of infective complications in surgical interventions of the gastro-enteric duct, as a coadjuvant in the therapy of hyperammonemias and in the reduction of the risk of acute events of hepatic encephalopathy.

Rifaximin is also useful in the articular fatigue syndrome, interstitial colitis, bacterial peritonitis and infections due to the chronic use of inhibitors of the proton pump.

For the aforesaid applications, the scarce absorption of rifaximin which is presently commercially available is a further advantage because it reduces the incidence of undesired effects such as, e.g., the selection of bacterial antibiotics-resistant strains and the risk of possible pharmacological interactions.

Rifaximin is presently commercially available in the form of tablets or capsules at doses comprised between 100 mg and 550 mg, or in preparations for pediatric use, or in ointments for treating topical infections.

Clinical studies have shown the efficacy of rifaximin in the treatment of pathologies with alterations of microbiota and of inflammatory bowel diseases, such as, for example, irritable bowel syndrome (IBS), small intestinal bacterial overgrowth (SIBO) and diverticular disease, Crohn's disease and pouchitis.

Rifaximin, administered with different therapeutic schemes with doses up to 1200 mg/day for a maximum duration of four weeks, induced significant improvements of IBS and SIBO symptoms as reported by Pimentel M. in Expert Opin. Investig. Drugs 2009; 18 (3), 349-358 and Prantera C. et al. in Aliment. Pharmacol. 23,1117-1125, 2006.

Among the bowel inflammatory diseases, a particular relevance is given to Crohn's disease, a pathology that may involve the whole alimentary tract, wherein the distal ileum and the colon are the most involved areas. The inflammatory process is typically discontinuous. The disease is characterized by a prolonged and variable course, by its systemic and perianal complications and by a relapsing tendency even after surgical and medical treatment.

Also with regard to this pathology, the use of rifaximin is reported, for example in, Shafran I. et al., Am. J. Gastroenterol., 2003, 98 (Suppl), S250 and Current Medical Research Opinion, 2005, 21(8), 1165-6, which report that, by using commercially available tablets containing 200 mg of rifaximin in the treatment of patients affected by Crohn's disease, a clinical efficacy in the induction of a remission of the disease was observed. Rizzello F. et al., Gut, 2000, 47, Suppl.3, A12 reports the use of 200 mg tablets of rifaximin in the prevention of a relapse.

Latella et al., in Int. J. Colorectal Dis., 2003, 18, 55-62 report the efficacy of the use of commercially available tablets of rifaximin for improving the symptoms of patients affected by a non complicated diverticular disease.

The therapeutic efficacy of rifaximin in treating hepatic encephalopathy is reported in Bass et al., New Eng. J. Med., 2010, 362, 1071-1081.

In order to improve the therapeutic efficacy of rifaximin in treating inflammatory bowel diseases, pharmaceutical compositions in the form of microgranules having a size smaller than 300 microns and coated by a gastroresistant film, as described in IT1362820, have been recently proposed.

These microgranules satisfy the requirement of releasing the antibiotic in the intestine only, and due to their large surface area and the composition of the gastroresistant coating with polymers that are bioadhesive on the mucosa, increase the contact of the active principle with the intestinal mucosa.

The preparation of pharmaceutical compositions comprising rifaximin in gastroresistant microgranules preferably for use in aqueous solutions for oral administration is described in International application WO 2006/094737 and U.S. Patent Application having publication no. US 20090011020 It is also reported in WO 2006/094737 that this preparation can be useful for treating inflammatory bowel diseases.

Prantera C. et al., in Aliment. Pharmacol. Ther., 2006, 23, 1117-1125 describe the results obtained in treating Crohn's disease with rifaximin in gastroresistant microgranules prepared according to WO 2006/094737, for aqueous suspensions, wherein a 12-week treatment with a 800 mg dose twice a day induced clinical remission in 52% of patients. Clinical remission rate reached 63% in the subgroup of patients with elevated baseline C-reactive protein values.

The use of rifaximin in microgranules for suspensions gave better and unexpected results compared to that which was reported by Shafran I. et al., Am. J. Gastroenterol., 2003, 98 (Suppl), S250 and Current Medical Research & Opinion 2005, 21(8), 1165-6, wherein, using 200 mg tablets of rifaximin three times a day, after a 12-week treatment remission of the disease was reported in 52% of the cases. To obtain a higher efficacy, the average duration of the treatment must be prolonged to at least 16 weeks.

When comparing the results obtained by treating patients with commercially available tablets compared to treating patients with gastroresistant microgranules for aqueous suspensions, it must also be noted that in the clinical study reported by Shafran, Am. J. Gastroenterol., 2003, 98 (Suppl), S250 and Current Medical Research and Opinion 2005, 21(8), 1165-6, 27.6% of patients also used corticosteroids as a concomitant therapy, whereas corticosteroids were excluded in the study carried out by Prantera, Aliment. Pharmacol. Ther., 2006, 23,1117-1125.

Shafran et al., Dig. Dis. Sci., 2010, 55(4), 1079-84 also report results of the use of rifaximin in 200 mg tablets three times a day in 68 patients affected by Crohn's disease, who maintained various concomitant therapies including corticosteroids, other antibiotics besides rifaximin, the so-called biological products, immunomodulators and anti-diarrheic agents for a therapy duration comprised between 0.4 and 134.9 weeks (average duration value 16.6 weeks). This study reports that the clinical remission of the disease was obtained in 65% of patients.

Although the aforesaid treatments described by Shafran were quite heterogeneous, it can be concluded that the results reported by Prantera, Aliment. Pharmacol. Ther., 2006, 23, 1117-1125, are indicative of therapeutic efficacy because the clinical remission is obtained in a shorter period of treatment, with a lower frequency of daily administrations and with no concomitant therapies with corticosteroids, other antibiotics and/or biological products.

WO2006/094737 describes the preparation of gastroresistant microgranules of rifaximin and discloses the preparation of compositions in suspensions, tablets and capsules. Moreover, it describes the use of formulations in microgranules for aqueous suspensions at doses up to 1600mg/day in patients affected by Crohn's disease, but without reporting about the efficacy and the safety at higher doses and about the duration of the remission of the disease after the end of the treatment.

Furthermore, WO2006/094737 describes possible pharmaceutical forms using microgranules, as tablets, capsules, packets, and states that the microgranules can contain α, β, γ, δ and ε polymorphs of rifaximin.

Rifaximin may also exist in hydrate and/or solvate forms. Solvates of rifaximin in which the form of the rifaximin is stabilized by the solvate is described in WO2008/029208 (also published as U. S. Patent Application Pub. No. 20100010028).

It is the object of this invention to provide more efficacious pharmaceutical forms for treating inflammatory diseases susceptible to treatment with rifaximin.

### Summary of the Invention

It has been unexpectedly found that by selecting to use tablets among the possible pharmaceutical forms, and by selecting among all polymorphous forms of rifaximin, to use hydrate or solvate form of rifaximin β polymorph, a more efficacious pharmaceutical form for treating inflammatory diseases is obtained as shown in Table 1.

This result is unexpected and surprising because tablets, among all pharmaceutical forms, the one wherein an active principle is less rapidly available in time, and the hydrate or solvate form of rifaximin β polymorph of rifaximin is the less bioavailable and the less soluble among all known polymorphous forms of rifaximin. The combination of these two factors makes the increased therapeutic efficacy of the formulations of this invention in treating inflammatory diseases and in particular Crohn's disease surprising and unexpected.

The tablets of the invention are preferably manufactured such that the hydrate or solvate form of rifaximin β is maintained during the whole production process and during their storage. Moreover, the obtained tablets should preserve the microgranules gastroprotection; and contain varying amounts of hydrate or solvate form of rifaximin β, alone or in mixture with other polymorphous or amorphous forms of rifaximin, also at doses equal to or higher than those described in WO2006/094737A1, i.e. 1600 mg/day; have an acceptable size; have a suitable hardness to be easily handled in the production and packaging process without danger of breaking or crumbling; have a disaggregation time sufficiently rapid to allow the release of the active principle in the intestine; and have a dissolution profile of the active principle equivalent to the one obtained with the aqueous suspensions of gastroresistant granules described in WO2006/094737A1. Furthermore, the tablets of the invention should be stable over time to be easily administered and acceptable to patients for being used in bowel infective and inflammatory diseases.

Moreover, although rifaximin exists in various polymorphous, enantiomeric, hydrated, solvated and amorphous forms, as described, for example in WO2005/044823, WO2006/094662, US2009082558, WO2008/035108, WO2008/155728, WO2009/10873001, U.S. Patent No. 7,045,620, WO2008/029208 (also published as U. S. Patent Application Pub. No. 20100010028 and in Crist. Eng. Comm., 2008, 10, 1074-1081 and the different forms may be characterized by different chemical-physical properties and bioavailability (see for example, U.S. Patent Application publication No. 20080132530), the pharmaceutical preparations of this invention for use in tablets prepared with gastroresistant microgranules maintain the form of rifaximin comprising, in particular, the hydrate or solvate form of rifaximin β, alone or in mixture with other rifaximin forms. WO2008/029208 (also published as U. S. Patent Application Pub. No. 20100010028 describes polyol solvates of rifaximin that stabilize the polymorphic form of rifaximin, particularly the β form, which are also suitable for use with this invention. Other solvates of rifaximin besides with polyols are also suitable for use with this invention.

In a preferred embodiment, the hydrate or solvate form of rifaximin β form is at least half of the total amount of rifaximin present in the tablet formulation. In other embodiments the hydrate or solvate form of rifaximin β is at least 70%, 75%, 80%, 85%, 90%, 95% or 100% of the total amount of rifaximin present in the tablet formulation.

Furthermore, the pharmaceutical form in tablets comprising the hydrate or solvate form of rifaximin β, alone or in mixture with other crystalline or amorphous forms of rifaximin, in gastroresistant microgranules prepared as described in WO2006/094737, comprises pharmaceutically acceptable extra-granular excipients, wherein the total amount of all extra-granular excipients preferably does not exceed 30% by weight of the tablet. In particular, the excipients comprise disintegrants, such as for example sodium carboxymethylcellulose also called carmelose, cross-linked sodium carboxymethylcellulose also called croscarmelose, or sodium starch glycolate and lubricants, such as for example magnesium or calcium stearate, sodium stearyl fumarate, vegetable hydrogenated oils, mineral oils, polyethylene glycols, sodium lauryl sulfate, glycerides, sodium benzoate.

The solid composition is characterized by the fact that it is bioadhesive, comprising microgranules coated by compounds having bioadhesive properties.

The tablets can also contain diluents, such as, for example, cellulose, microcrystalline cellulose, calcium phosphate, starch, kaolin, hydrated calcium sulfate, calcium carbonate, lactose, sucrose, glucose, glucans, xyloglucans, provided that the sum of the weight of the excipients is always lower than about 30% by weight of the tablets, more in particular comprised between 5.0% and 20.0%.

Optionally, the tablets comprising microgranules of rifaximin may also be coated with a filmogenous coating. It is not necessary a gastroresistant film coating and even a division of the tablets maintains the gastroresistance.

A desirable and advantageous characteristic of the new composition of gastroresistant tablets of the invention comprising hydrate or solvate form of rifaximin β, alone or in mixture with other amorphous, hydrated or solvated crystalline forms is that, in some embodiments, it is not necessary for the tablets to contain ligand and/or glidant excipients, commonly used in the state of the art.

The tablets comprising hydrate or solvate form of rifaximin β, alone or in mixture with other rifaximin forms, are stable over three years at 25°C and over six month at 40°C, have suitable hardness, friability and disaggregation properties and keep their gastroresistant characteristic even when the tablets are divided. With the gastroresistant microgranules containing hydrate or solvate form of rifaximin β, alone or in mixture with other rifaximin forms, tablets containing varying amounts of rifaximin can be prepared since the microgranules can be mixed with one or more excipients known in the art until reaching the desired weight of the tablet as an alternative to their possible division.

The tablets prepared with the microgranules containing hydrate or solvate form of rifaximin β, alone or in mixture with other forms of rifaximin can contain varying amounts of rifaximin comprising between 100 and 800 mg, inclusive, and are such that they can be usefully administrated for indications requiring high doses, and in particular doses higher than 1600 mg per day, in patients affected by bowel infective and inflammatory diseases, such as, but not limited to, e.g. Crohn's disease, with demonstrated therapeutic efficacy, safety and tolerability.

The tablets prepared according to the present invention comprising hydrate or solvate form of rifaximin β, alone or in mixture with other rifaximin forms, in gastroresistant microgranules, like the microgranules described in WO2006/094737, showed a higher therapeutic efficacy in treating bowel inflammatory diseases, like e.g. Crohn's disease, if compared to the results obtained by administering the same microgranules in aqueous solution.

As an example, Table 1 reports the results obtained from the clinical study described in Example 11, wherein the percentage of patients obtaining complete clinical remission of the disease after the administration of the tablets comprising hydrate or solvate form of rifaximin β in gastroresistant microgranules compared to the patients who received a suspension of the same microgranules prepared as reported in Example 7 of WO2006/094737.

**Table 1**

| | 400 mg Rifaximin tablets prepared as in Example 2 *(Example 11 of the present invention)* | Rifaximin microgranules in aqueous suspension ***(Example* 7 *of*** WO2006/094737***)*** |
|---|---|---|
| Dose /day | 1600 mg | 1600 mg |
| Treatment duration | 12 weeks | 12 weeks |
| Clinical remission percentage | 62.2% | 51.9% |

The new tablet composition of the invention comprising hydrate or solvate form of rifaximin β and the method for their preparation are such to confer unexpected therapeutic advantages if compared to the rifaximin prepared in microgranules for aqueous suspensions.

This invention provides the use of a pharmaceutical solid composition hydrate or solvate form of rifaximin β, alone or in a mixture with other crystalline, hydrate, solvate or amorphous forms of rifaximin, present in gastroresistant microgranules, for the administration to patients in need thereof of a daily dosage higher than 2000 mg for a period of treatment longer than 7 days which does not induce rifaximin accumulation in plasma. In a study of the present invention, patients received 1200 mg of rifaximin prepared as in Example 2 on day 1; 2400 mg on days 2, 3, 4, 5, 6; and 1200 mg on day 7. The steady state plasma concentration was reached by day 3, since the plasma concentration at the pre-dose of days 3 and 5 did not increase compared with those at pre-dose of day 7. In an embodiment of the invention, the rifaximin pharmaceutical composition provides a maximum plasma concentration of rifaximin (Cmax) value less than 7 ng/ml after five or seven days of treatment at a dosage higher than 2000 mg/day. In another embodiment the use of a pharmaceutical composition according to the invention provides an AUC₀₋₂₄ₕ value that is less than 48 ng·h/ml after five or seven day of treatment at a dosage higher than 2000 mg/day.

Another embodiment of the invention provides the use of the pharmaceutical composition according to the invention wherein the Cmax is reached within in less than 4 hours after five or seven days of treatment at a dosage higher than 2000 mg/day. The invention also provides for the use of a pharmaceutical solid composition comprising hydrate or solvate form of rifaximin β, alone or in a mixture with other crystalline, hydrate, solvate or amorphous forms in gastroresistant microgranules, characterized in that they are efficacious at a daily dose of from 800 mg to 2400 mg for the treatment of intestinal infective and inflammatory disease, such as for example Crohn's disease, without concomitant therapy.

In other embodiments of the invention, pharmaceutical compositions according to the invention for use in methods of treating persons in need of treatment having intestinal infective and/or inflammatory disease, such as for example, Crohn's disease are provided. In an embodiment, the method wherein the compositions of the invention are used, comprises the administration to patients in need thereof, a daily dosage of a pharmaceutical solid composition comprising hydrate or solvate form of rifaximin β, alone or in a mixture with other crystalline, hydrate, solvate or amorphous forms of rifaximin, in gastroresistant microgranules, wherein the dosage is higher than 2000 mg per day for a period of treatment longer than seven days and does not induce rifaximin accumulation in plasma. The methods of the invention may be used to achieve the additional uses of the invention described above.

This invention also includes pharmaceutical compositions comprising hydrate or solvate form of rifaximin β, alone or in a mixture with other crystalline, hydrate, solvate or amorphous forms of rifaximin present in gastroresistant microgranules, for the administration to patients in need thereof a daily dosage higher than 2000 mg for a period of treatment longer than seven days does not induce rifaximin accumulation in plasma.

### Detailed description of the embodiments

The object of the invention is obtained through solid compositions in the form of gastroresistant tablets prepared with gastroresistant microgranules having a size of 300 microns or less, comprising hydrate or solvate form of rifaximin β and pharmaceutically acceptable excipients.

The solid compositions in the form of tablets can comprise an amount of hydrate or solvate form of rifaximin β, alone or in mixture with other forms of rifaximin between 100 and 800 mg of hydrate or solvate form of rifaximin β, are able to prevent the release of rifaximin at pH values between about 1.5 and about 4.0 and allow the release of rifaximin at pH values comprised between about 5.0 and about 7.5.

The solid compositions according to the invention obtained with gastroresistant microgranules comprising hydrate or solvate form of rifaximin β, alone or in mixture with other rifaximin forms, have unexpected characteristics of therapeutic efficacy and adequate hardness, disaggregation, homogeneity and stability over time, also allowing the preparation of divisible tablets while maintaining their gastroresistance.

The compositions in the form of tablets containing hydrate or solvate form of rifaximin β, alone or in mixture with other forms of rifaximin, in gastroresistant microgranules having bioadhesive properties, optionally comprise pharmaceutically acceptable extragranular excipients in an amount such that it does exceed 30%, preferably 5-20%, by weight of the tablet.

These extragranular excipients comprise: disintegrants, such as, for example, sodium carboxymethylcellulose also called carmelose, cross-linked sodium carboxymethylcellulose also called croscarmelose, or sodium starch glycolate, and lubricants, such as, for example, magnesium or calcium stearate, sodium stearyl fumarate, vegetable hydrogenated oils, mineral oils, polyethylene glycols, sodium lauryl sulfate, glycerides, sodium benzoate.

The tablets may also comprise diluents such as, for example, cellulose, microcrystalline cellulose, calcium phosphate, starch, kaolin, di-hydrated calcium sulfate, calcium carbonate, lactose, saccharose, glucose, polysaccharides, glucans, xyloglucans. The tablets may also comprise colorants, flavoring agents, anti-oxidants, sweeteners.

A relevant aspect of the invention is that the total sum of all extragranular excipients added to gastroresistant microgranules, in any possible ratio, does not exceed 30% by weight of the tablet, and preferably comprised between 5% and 20%.

In another aspect of the invention tablets comprising hydrate or solvate form of rifaximin β, alone or in mixture with other forms of rifaximin, in gastroresistant microgranules, are provided have a disaggregation time sufficiently long to so that the preparation is suitable for the release of the active principle rifaximin in the intestinal tract. Preferably, the disaggregation time is at least 8, 8.5 or 9 minutes.

Another aspect of the invention provides tablets comprising hydrate or solvate form of rifaximin β, alone or in mixture with other forms of rifaximin, in gastroresistant microgranules, having a hardness of between 180 and 215 Newton, so that the tablet can be easily handled during the production and packaging process in blisters or bottles with no particular further precautions.

Another aspect of the invention are tablets comprising hydrate or solvate form of rifaximin β, alone or in mixture with other forms of rifaximin, in gastroresistant microgranules, with friability values of between 0.0% and 0.1% so that the tablet can be easily handled during the production process.

The compositions according to the invention allow the release in the intestinal lumen of the entire amount of the dose of hydrate or solvate form of rifaximin β and of other possible rifaximin forms included in the formulation.

Another aspect of the invention provides pharmaceutical compositions useful for treating/improving subjects affected by bowel diseases such as, but not limited to, infective bowel pathologies caused by non entero-invasive bacteria (traveler's diarrhea, enteritis, dysentery), bowel inflammations such as, for example, Crohn's disease, ulcerous recto-colitis, irritable bowel syndromes (IBS), paucities, small intestine bacterial overgrowth (SIBO), diverticular syndromes; pathologies directly or indirectly deriving from bowel infections, such as, for example, hepatic encephalopathy, or usable in the pre- and post-surgical prophylaxis of bowel infections.

"Treating," "treatment," or "therapy" of a disease or disorder means slowing, stopping, or reversing progression of the disease or disorder, as evidenced by a reduction or elimination of either clinical or diagnostic symptoms. These terms do not necessarily mean total cure. Any alleviation of any undesired signs or symptoms of the disease to any extent or the slowing down of the progress of the disease can be considered treatment.

Furthermore, treatment may include acts that may worsen the patient's overall feeling of well being or appearance. Treatment may also include lengthening the life of the patient, even if the symptoms are not alleviated, the disease conditions are not ameliorated, or the patient's overall feeling of well being is not improved.

The tablet compositions according to the invention can comprise pharmaceutically acceptable excipients and/or other active principles administered in association with other active principles without dangerous interactions.

The solid tablet compositions of the invention can be included in kits comprising the tablets obtained with microgranules comprising therapeutically effective amounts of hydrate or solvate form of rifaximin β, alone or in association with other forms of rifaximin for treating patients with bowel diseases and instructions for using said tablets.

Another aspect of the invention is a method of treating and/or preventing bowel infective and inflammatory diseases or for the prophylaxis of bowel diseases in a person in need thereof comprising administering a pharmaceutical composition in the form of tablets comprising hydrate or solvate form of rifaximin β, alone or in association with other rifaximin forms, in gastroresistant microgranules, wherein one or more tablets are administered to achieve a therapeutic effective amount and wherein said therapeutically effective amount may include doses higher than 1600 mg daily with demonstrated efficacy, safety and tolerability. In one embodiment of the invention the dosage may be up to and including 2400 mg per day. Other doses suitable for use according to the invention may be 1700, 1800, 1900, 2000, 2100, 2200 and 2300 mg per day.

Another particular aspect of the invention is the provision of pharmaceutical compositions according to the invention for use in a method of treating a patient with a gastroresistant formulation comprising administering to a patient in need thereof a pharmaceutical formulation comprising microgranules comprising an amount of hydrate or solvate form of rifaximin β, alone or in association with other rifaximin forms, efficacious for the remission of bowel infections and/or inflammations at the end of the treatment with said pharmaceutical composition. Such infections and/or inflammations may include, but not be limited to those selected from the group of, irritable bowel Syndrome (IBS), Crohn's disease, irritable bowel syndromes with associated uncontrolled diarrheas (dIBS), hepatic encephalopathy, diarrheas, traveler's diarrhea, ulcerous colitis, enteritis, small intestine bacterial growth, chronic pancreatitis, pancreatic insufficiency, colitis and diverticular syndromes. The method according to the invention may also be used to provide a method for antibacterial prophylaxis before a surgical intervention. An advantage of the method according to the invention is that at the end of the administration for a period of treatment comprising between 10 and 20 weeks, no toxic effects are observed for the patient and there is good tolerability.

Another aspect of the invention includes using the solid composition of the invention comprising microgranules comprising hydrate or solvate form of rifaximin β, alone or in association with other rifaximin forms, to maintain the remission of the bowel inflammation for a period longer than three months after the end of the pharmacological treatment with rifaximin in gastroresistant composition.

Another aspect of the invention is the formulations of the invention comprising hydrate or solvate form of rifaximin β, alone or in association with other rifaximin forms in gastroresistant microgranules, for use in a method comprising administering to a patient in need thereof doses of rifaximin higher than 1600 mg/day, including 2400 mg/day and doses in between as stated above, to obtain remission of the disease in the patient affected by inflammatory bowel diseases and in particular by Crohn's disease, having values of C-reactive protein comprised between 5 and 10 mg/l.

The composition and methods of the invention provide demonstrated efficacy of the pharmaceutical composition of the invention formulated in tablets comprising gastroresistant microgranules of hydrate or solvate form of rifaximin β, alone or in association with other rifaximin forms, for the treatment and the remission of moderately active Crohn's disease.

Hydrate or solvate form of rifaximin β, alone or in association with other forms of rifaximin, at a dosage from 100 to 800 mg in the form of tablets comprising rifaximin in gastroresistant microgranules can be administered at a dosage corresponding to one dose, one, two or three times a day; to two doses, one, two or three times a day; or to three doses, one or two times a day; or to four doses, one or two times a day, with no relevant limitation or side effect or interaction with food or other drugs which must be concomitantly taken.

An advantage of the invention is that the administration of tablets comprising hydrate or solvate form of rifaximin β, alone or in association with other rifaximin forms, in the form of gastroresistant microgranules may be repeated at any dosage for several treatment cycles. Such treatment cycles may provide for the administration of rifaximin up to and including at least 10 or 20 weeks of treatment, or as needed to reduce or eliminate symptoms associated with bowel disease suitable for treatment with rifaximin such as those stated above, including Crohn's disease.

Hydrate or solvate form of rifaximin β, alone or in association with other rifaximin forms, in the composition in gastroresistant microgranules, can be taken alone or concomitantly with other drugs, such as, for example, 5-ASA, immunosupressants such as, for example, azathioprine, methotrexate, 6-mercaptopurine, corticosteroids, biological products such as infliximab, etanercept, adalimumab, anakirna, with little or no interaction.

The gastroresistant microgranules comprising hydrate or solvate form of rifaximin β, alone or in association with other rifaximin forms, can be prepared as described in Example 1. The coating of the microgranules comprises polymeric materials which are preferably insoluble at pH values between about 1.5 and 4.0 and which are soluble at pH values between 5.0 and 7.5. In general, the gastroresistant coating is made of any material which is insoluble in a pH range between 1.5 and 4.0 and which is soluble at higher pH values, preferably at pH values between 5.0 and 7.5. Examples of particular polymers which are suitable for use with this invention are chosen among the copolymers of acrylic acid, such as the methacrylic acid-ethyl acrylate copolymer 1:1 and the methacrylic acid-methyl methacrylate copolymer 1:2, polyvinyl acetate phtalate, hydroxypropylmethylcellulose phtalate, cellulose acetate phtalate, commercially available, for instance, with the trademarks Kollicoat®, Eudragit®, Aquateric®, Aqoat®.

The amount of gastroresistant material used to make the microgranules for use with the invention is preferably between 10% and 60%, more preferably between 20% and 40%, if compared to the total weight of the gastroresistant granule. The gastroresistant coating which is applied on the active principle in microgranules can optionally also contain plasticizers, diluents, antiadherents, antiagglomerants, glidants, defoamers, colorants and antioxidants.

The components which are used for coating microgranules, are solubilized using organic solvents or kept in an aqueous suspension. The solutions or suspensions of coating material are applied by nebulization on powders or granules or microgranules which are kept moving inside a coating pan or in air suspension in fluid bed apparatuses during the application process.

Non-limiting examples of organic solvents that may be used to solubilize the coating material are methylene chloride, methyl acid, isopropyl alcohol, triethyl acetate and ethyl alcohol.

As an alternative, the gastroresistant polymeric material can be applied through aqueous suspensions, which is the preferable technique because it does not require the use of solvents with the relating toxicological and safety concerns.

The gastroresistant microgranules can also be prepared with other processes known to those in the pharmaceutical arts. Such techniques may include, for example, granulating the active principle rifaximin together with diluents, glidants and ligands, and by submitting the dried and sieved microgranules to the successive coating process with a gastroresistant coating.

Another system which can be used for the preparation of the microgranules involves the application of rifaximin by means of a ligand compounds selected from the group consisting of cellulose, cellulose derivatives, starches, potatoes starch, corn starch, gums, synthetic gum, polyvinylpyrrolidone, sodium carboxymethyl cellulose, cellulose microcrystalline, hydroxypropylcelllose, hydroxyethylcellulose, hydroxypropylmethylcellulse, ethylcellulose, polyethylene glycol, gelatin, polypropylene glycol, alginic acid, alginate salts, sugars, and combinations thereof to microcrystalline cellulose granules having a diameter between about 100 and 200 microns and submitting the resulting microgranules to the successive coating process with the gastroresistant film.

Preparations of the gastroresistant tablets according to the invention are described wherein specific polymorphous forms of rifaximin can be included in the gastroresistant microgranules. The processes for the preparation of the gastroresistant microgranules preferably includes the particular conditions, as described in Example 1, incorporating the hydrate or solvate form of rifaximin β.

Rifaximin β may be prepared by methods known in the art and as described for example in U.S. Patent No. 7,045,620 and U.S. patent application publication no. US20080262220. If hydrate or solvate form of rifaximin β is prepared from α rifaximin, one source of polymorph α rifaximin is the rifaximin which is currently approved and commercialized for clinical use in Europe and sold as Normix®. Examples of solvates of rifaximin β that are also suitable for use with this invention are described in WO2008/029208 (also published as U. S. Patent Application Pub. No. 20100010028 which describes polyol solvates of rifaximin that stabilize the polymorphic form of rifaximin, particularly the β. Other solvates of rifaximin besides with polyols are also suitable for use with this invention as well.

The gastroresistant microgranules have bioadhesive properties, which means that they can adhere to the mucosa.

Examples of polymers and oligomers or their mixtures which can be included in microgranules are pectins, zeins, casein, gelatin, albumin, collagen, kitosan, oligosaccharides and polysaccharides such as, for example, cellulose, dextran, polysaccharides from tamarind seeds, xanthan gum, Arabic gum, hyaluronic acid, alginic acid, sodium alginate.

When the bioadhesive is a synthetic polymer, the polymer is chosen among polyamides, polycarbonates, polyalkylenes, polyalkylene glycols, polyalkylene oxides, polyalkylene terephthalates, polyvinyl alcohol, polyvinyl ethers, polyvinyl esters, polyvinyl pyrrolidone, polysiloxanes, polyurethanes, polystyrenes, polymers of acrylic acid and methacrylate esters, copolymer of methacrylic acid-ethyl acrylate, polyactides, polybarbituric acids, polyanhydrides, polyorthoesters and mixtures thereof. Other useful polymers are methyl cellulose, ethyl cellulose, hydroxy propyl cellulose, hydroxy butyl methylcellulose, cellulose acetate, cellulose propionate, cellulose acetate butyrate, carboxy methyl cellulose, cellulose triacetate, cellulose sulfate sodium salt, polymethyl methacrylate, poly-isopropyl methacrylate, poly-isobutyl acrylate, poly(octadecyl acrylate), polypropylene, polyethylene glycol, polyethylene oxide, polyethylene terephthalate, polyvinyl acetate, polyvinyl chloride, polystyrene, polyvinyl pyrrolidone, polyvinyl phenol and mixtures thereof.

Another group of polymers useful for obtaining the bioadhesiveness are polymers having a branching group comprising at least one bound hydrophobic group, wherein the hydrophobic groups are generally non polar groups. Non-limiting examples of such hydrophobic groups include alkyl, alkenyl and alkynyl groups. Preferably, the hydrophobic groups are chosen to increase the bioadhesiveness of polymers. Other polymers are characterized by hydrophobic branching with at least a hydrophilic group, such as carboxy acids, sulphonic acids and phosphonic acids, neutral and positively charged amines, amides and imines, wherein the hydrophilic groups are such that they increase the bioadhesiveness of the polymer.

According to a preferred method, the mixture containing the compounds suitable to form the coating is prepared by suspending the various components in demineralized water and by homogenizing the aqueous suspension with a high speed stirring system, preferably a Ultra Turrax homogenizer, in order to obtain a homogeneous suspension containing between 15% and 30% of solid particles. The application of the gastroresistant coatings to the pharmaceutical forms may then be carried out by means of coating pans or by fluid bed coating technology, for example operating under the conditions reported in Table 2 of Example 1, using the excipients listed in Table 3.

For the production of the tablets, pharmaceutically acceptable extragranular excipients may be added to the gastroresistant microgranules in a weight amount preferably lower than 30%, and more in preferably between 5% and 20%, compared to the total weight of the finished tablet.

The extragranular excipients comprise disintegrants, which may be chosen from among sodium carboxy methyl cellulose, also called sodium carmelose, cross-linked sodium carboxy methyl cellulose, also called croscarmelose, polyvinyl pyrrolidone, also called povidone, cross-linked polyvinyl polypirrolidone, also called crospovidone, starch, pre-gelatinized starch, silica and lubricants chosen among magnesium or calcium stearate, sodium stearyl fumarate, vegetable hydrogenated oils, mineral oils, polyethylene glycols, sodium lauryl sulfate, glycerides, sodium benzoate.

The tablets of the invention may also comprise diluents chosen among from among, for example, cellulose, microcrystalline cellulose, calcium phosphate, starch, kaolin, di-hydrated calcium sulfate, calcium carbonate, lactose, saccharose, glucose, sorbitol and mannitol. Colorants, flavoring agents, anti-oxidants, and sweeteners may also optionally be added to the tablet formulations.

One or more disintegrants, one or more lubricants and, possibly, one or more diluents, can optionally be used in the production of tablets.

The mixture comprising microgranules of hydrate or solvate form of rifaximin β, alone or in mixture with other rifaximin forms, disintegrants, lubricants and, optionally, diluents, colorants, anti-oxidants and sweeteners, is placed in a compressing machine, already known in the art, provided with suitable molds for preparing tablets with the desired shape and size.

The compositions in the form of tablets can be coated with a mixture constituting the coating, wherein the coating comprises, for example, talc, cellulose derivatives, anti-oxidants, salts, colorants, flavoring agents.

According to a preferred embodiment of the invention, the amount of disintegrants contained in the tablets varies between about 3% and 8% by weight; the amount of lubricants varies between about 2% and 3% by weight, and the amount of diluents varies between about 0% and 10% by weight of the tablet.

As stated above, the tablets can be formulated to contain variable amounts of hydrate or solvate form of rifaximin β or in association with other rifaximin forms according to the amount of incorporated rifaximin containing microgranules.

Examples 2-9 describe several useful compositions for the preparation of tablets comprising gastroresistant microgranules comprising hydrate or solvate form of rifaximin β, characterized in that they have an amount by weight of extragranular excipients less than 20% of the weight of the tablet.

Example 2 describes the preparation of tablets wherein a disaggregant such as, for example, sodium croscarmelose, and a lubricant such as, for example, magnesium stearate, and a diluent such as, for example, microcrystalline cellulose are added to the gastroresistant microgranules comprising rifaximin, prepared as in Example 1, wherein the sum of the components is less than 10% by weight of the tablet. The preparation of the obtained tablets does not modify the polymorphous form of rifaximin contained in the gastroresistant microgranules prepared as in Example 1.

In particular, Table 5 reports the exact composition of a tablet containing 400 mg of rifaximin made as described in Example 2, characterized in that it contains the same proportions of gastroresistant microgranules and extragranular excipients of Table 4 and has a coating wherein the total weight is less than 710 mg.

In this particular embodiment of the invention, the obtained gastroresistant tablets are characterized by the fact that they maintain the form of rifaximin contained in gastroresistant microgranules, have a hardness value of 198 Newton, a disaggregation time of 8.5 minutes and are not friable.

Example 3 describes the preparation of tablets wherein a disaggregant, in this example, sodium starch glycolate, and a lubricant, in this example, magnesium stearate, are added to gastroresistant microgranules comprising rifaximin, prepares as in Example 1, wherein the sum of the two components is less than 7% by weight of the tablet.

Example 4 describes the preparation of tablets wherein a disaggregant, in this example, sodium starch glycolate, a lubricant, in this example, magnesium stearate and a diluent, in this example, microcrystalline cellulose are added to gastroresistant microgranules comprising rifaximin, prepared as in Example 1, wherein the sum of the components is less than 6% by weight of the tablet.

Example 5 describes the preparation of tablets comprising rifaximin, in microgranules, prepared as in Example 1, wherein a disaggregant, in this example, sodium starch glycolate and a lubricant, in this example, magnesium stearate, are added to gastroresistant microgranules comprising a greater quantity of rifaximin than that present in Example 3, wherein the sum of the two components is less than 7% by weight of the tablet.

Example 6 describes the preparation of tablets wherein a disintegrant, in this example, sodium starch glycolate, a lubricant, in this example, magnesium stearate and a diluent, in this example, microcrystalline cellulose are added to gastroresistant microgranules comprising rifaximin prepared as in Example 1, wherein the sum of the extragranular excipients is less than 12% by weight of the tablet.

Example 7 describes the preparation of tablets wherein a disaggregants, in this example, sodium starch glycolate and a lubricant, in this example, magnesium stearate and a mixture of microcrystalline cellulose based diluents are added to gastroresistant microgranules comprising rifaximin prepared as in Example 1, wherein the sum of the components of extragranular excipients is less than 16% by weight of the tablet.

Example 8 describes the preparation of tablets wherein a disaggregant, in this example, sodium starch glycolate and a lubricant, in this example, magnesium stearate and a mixture of microcrystalline cellulose based diluents are added to gastroresistant microgranules comprising rifaximin prepared as in Example 1, wherein the sum of the components of extragranular excipients is less than 17% by weight of the tablet.

Example 9 describes the preparation of tablets wherein a disaggregant, in this example, sodium croscarmelose, a lubricant, in this example, magnesium stearate and a diluent, in this example, microcrystalline cellulose are added to gastroresistant microgranules comprising rifaximin prepared as in Example 1, wherein the sum of the components of extragranular excipients is less than 12% by weight of the tablet.

The tablets can be obtained in shapes such that they can be divided into smaller dosage forms, such as for example, two, three or four parts, wherein the division allows a further dosage variability without losing the gastroresistance.

The tablets of the invention are stable for a period longer than 12 months at temperatures between 25°C and 30°C and for a period longer than 6 months at 40°C.

The safety determination of rifaximin in the composition in the form of tablets prepared as in Example 2 has been evaluated as described in Example 10 wherein 18 healthy volunteers received a dosage higher than 1000 mg daily of rifaximin by administration of rifaximin tablets before and after meals. The obtained results show that no adverse events relating to the drug administration or other contraindications to its use have been reported.

The safety of rifaximin in the composition in the form of tablets comprising rifaximin, prepared as in Example 2, in gastroresistant microgranules has been evaluated also in patients affected by inflammatory bowel disease, for example Crohn's disease. In particular, as described in Example 10, 18 fasted patients with active Crohn's disease were administered a dosage higher than 1000 mg /day of rifaximin tablets prepared as in Example 2. The results obtained show that no adverse events related to the drug administration or other contraindications to its use have been reported.

Example 11 describes clinical trial, wherein 402 patients affected by intestinal inflammation with a CDAI value between 220 and 400 were treated with the composition of the invention in a form of tablets comprising rifaximin, prepared as in Example 2, in comparison to placebo for a period of time of 12 weeks.

The patients of the clinical trial were divided into four groups:
Group A: the patients were administered one tablet of 400 mg rifaximin prepared as in Example 2, comprised in gastroresistant microgranules, twice a day for a total dosage of 800 mg of rifaximin;
Group B: the patients were administered two tablets of 400 mg rifaximin prepared as in Example 2 comprised in gastroresistant microgranules 2 times a day for a total dosage of 1600 mg of rifaximin;
Group C: the patients were administered three tablets of 400 mg rifaximin prepared as in Example 2 comprised in gastroresistant microgranules, 2 times a day for a total dosage of 2400 mg of rifaximin;
Group D: 99 patients were administered three placebo tablets 2 times a day.

All the patients maintained their concomitant therapy, if in use before the start of clinical trial.

Table 13 reports the results of a clinical trial in patients with Crohn's disease, to whom the new composition in the form of tablets, described in Example 2, was administered at a daily dosage of from 800 mg/day to 2400 mg/day and placebo.

From the results reported in Table 13, the tablets comprising gastroresistant microgranules of rifaximin prepared as in Example 2 are more efficacious than placebo. As can be seen in Table 13, compared to placebo: a greater percentage of the patients had clinical remission at the end of treatment; a greater percentage of the patients had a clinical response at the end of treatment; a greater percentage of the patients maintained the clinical response after 12 weeks after the end of the treatment; the number of days to obtain the clinical remission was reduced; and the percentage of the patients which did not respond to the clinical treatment was reduced as well.

The evaluation of adverse events and adverse reactions in patients treated with tablets comprising gastroresistant microgranules of rifaximin prepared as in Example 2in comparison to patients who received placebo is described in Table 14. The evaluation shows that there is not any difference in the number and severity of adverse events between several rifaximin dosages in comparison to the placebo. These results confirm that tablets comprising gastroresistant rifaximin at a daily dosage from 800 mg/day to 2400 mg/day are safe.

Example 12 describes the clinical remission of Crohn's disease in patients with protein C reactive values greater than 5mg/l administered high daily dosages of tablets comprising gastroresistant microgranules of rifaximin prepared as in Example 2. Table 15 reports a higher percentage of patients who obtained clinical remission at the end of treatment for patients administered a dosage greater 1600 mg/day compared to patients administered placebo or dosages less than 1600 mg/day.

The Example 13 describes the evaluation of rifaximin accumulation in plasma after repeated administrations of gastroresistant tablets comprising rifaximin prepared as in Example 2, to 12 patients affected by mild-to-moderate active Crohn's disease.

Rifaximin prepared as in Example 2 in the form of gastroresistant tablets, was administrate to patients in a quantity corresponding to 1200 mg on day 1; 1200 mg twice a day, for a total dosage equal to 2400 mg/day on days 2, 3, 4, 5, 6; and 1200 mg on day 7, for a total dosage of 14400 mg of rifaximin in seven days.

The plasma concentration values of rifaximin at the pre-dose of the third and fifth days were comparable to the plasma concentration at the pre-dose of the seventh day, demonstrating that the steady state of rifaximin plasma concentration was reached.

The plasma concentrations of rifaximin were analyzed before the first dosage administration and until to 24 hours following the dosage administration during the first day of the treatment and up to 48 hours after the last dose during the seventh day of treatment. Plasma concentrations were also determined before dosage administration on the third and fifth days.

The pharmacokinetic parameters were determined, such as the maximum plasma concentration achieved (Cₘₐₓ), the area under the curve of plasma concentrations (AUCₗₐₛₜ) and the time to achieve the maximum concentration (Tₘₐₓ).

The Table 16 reports the Cmax, AUClast and Tmax values after administration of rifaximin the first and the seventh day. The results show that rifaximin is poorly absorbed in patients affected by mild-to-moderate active Crohn's disease, and even following repeated administration of rifaximin prepared as in Example 2 at high dosages is not accumulated in the plasma.

Example 14 describes the efficacy of rifaximin in the form of gastroresistant tablets in patients affected by moderate active Crohn's disease without concomitant treatment, who have been treated with a dosage of rifaximin prepared as in Example 2 equal to 800, 1600 and 2400 mg/day.

The patients are divided into four groups wherein:
Group A: have been treated with one 400 mg tablet of rifaximin prepared as in Example 2, administrated 2 times a day for a total dosage equal to 800 mg/day;
Group B: have been treated with two gastroresistant 400 mg tablets of rifaximin prepared as in Example 2, administrated two times a day, for a total dosage equal to 1600 mg/day;
Group C: have been treated with three 400 mg tablets of rifaximin prepared as in Example 2, administrated two times a day, for a total dosage equal to 2400 mg/day;
Group D: have been treated with gastroresistant tablet without rifaximin (placebo).

Table 17 shows the results wherein the patients treated with gastroresistant tablets of rifaximin have obtained a clinical remission greater than the patients treated with placebo. Particularly the group of patients treated with a dosage of rifaximin prepared as in Example 2 equal to 1600 mg/day show a statistically significant difference in clinical remission rate compared to placebo.

Ulcerative colitis and Crohn's disease are associated with increased risk of colorectal cancer (CRC). Oxidative stress due to the chronic inflammation associated with Intestinal Bowel Disease may be one of the causes of CRC in IBD patients as described by Eaden et al. n Gut, 2001, 48, 526-35 and by Roessner in Pathol. Res. Pract.2008, 204, 511-24.

The examination of faecal contents is a non-invasive approach to monitor the exposure of the colonic mucosa to mutagenic and genotoxic substances.

Single cell gel electrophoresis (Comet assay) has been successfully employed in nutritional intervention studies to monitor DNA damage in colon carcinoma cell lines as described for example by Venturi et al. in Carcinogenesis 1997, 18, 2353-9 and Klinder in Nutr. Cancer 2007, 57, 158-67. Example 15 describes the effect of rifaximin treatment on faecal water genotoxicity and DNA damage through Comet assay and it demonstrates that in patients who received rifaximin prepared as in Example 2, shown a significant reduction of strand breakage s induced by faecal water.

The following Examples describe the preparation of gastroresistant tablets comprising rifaximin prepared as in Example 2 and their use in the treatment of inflammatory bowel disease and are to be considered as a further illustration of the invention without any limitation.

### Example 1

### Preparation of Rifaximin in gastroresistant microgranules

In a fluid bed apparatus, Glatt GPC 30, with a Wurster system of 18 inches with a 1.8 mm spray jet, 25000 g of rifaximin powder and 125 g of Aerosil as fluidiser were loaded. Contemporaneously in a mixer under agitation a suspension was prepared using 48107 g of demineralised water, 9281g of methacrylic acid ethylacrylate copolymer marketed under the trademark KOLLICOAT® MAE 100 P, 1392 g 1,2 propandiol, 2475 g of talc, 557 g of titanium dioxide FU and 62 g of iron oxide E 172. The solid components of the suspension were homogeneously mixed in demineralised water with a high speed homogeniser (Ultra Turrax). The prepared suspension was loaded in the Wurster type apparatus with a peristaltic pump and nebulised, at a pressure between 1.0 and 1.5 bar, through the 1.8 mm nozzle on the mixture of rifaximin powder and Aerosil 200 maintained in suspension in the fluid bed by a warm air flow.

The applied conditions are described in Table 2:

**Table 2**

| Process parameters | Pre-warm phase | Application of coating solution | Drying |
|---|---|---|---|
| Air flow in entrance (m³/hour) | 400 ± 100 | 550 ± 100 | 350 ± 50 |
| Air temperature in entrance (°C) | 60 ± 2 | 60 ± 10 | 70 ± 2 |
| Product temperature (°C) | 32 | 25 - 27 | 30 ± 2 |
| Jet pressure (bar) (initial phase) | | 1-1.5 ± 0.1 | |
| Jet speed (g/min) | | 150 - 200 | |

The obtained microgranules were submitted to granulometry analysis by Light Scattering technology using a Malvern Mastersizer 2000 apparatus which result in greater than 91% of the microgranules having a dimension lower than 300 micron.

The microgranules composition comprising rifaximin is described in Table 3.

**Table 3**

| Composition | Amount (grams) | Amount (%) |
|---|---|---|
| Rifaximin | 25000 | 64.3 |
| Silica (Aerosil ® 200) | 125 | 0.3 |
| Methacrylic acid methylmethacrylate copolymer 1:1(Kollicoat® MAE 100P) | 9281 | 23.9 |
| 1,2 propandiol | 1392 | 3.6 |
| Talc | 2475 | 6.4 |
| Titanium dioxide FU | 557 | 1.4 |
| Iron oxide E172 | 62 | 0.2 |

The microgranules obtained were analyzed by X-ray diffraction. The diffractograms show that the rifaximin has peaks characteristic of the β form, as identified in Crist. Eng. Comm. 10, 1074-1081 (2008

### Example 2

### Preparation of tablets comprising rifaximin in gastroresistant microgranules -Composition 1-

The microgranules comprising rifaximin prepared as described in Example 1, were mixed with croscarmelose (Ac-Di-Sol®) and microcrystalline cellulose (Avicel PH 102®) previously sieved on a 0.8 mm mesh sieve. Magnesium stearate was then added to the resulting mixture. The resulting composition is described in Table 4.

**Table 4**

| Composition | Amount (mg) | Component percentage (p/p) |
|---|---|---|
| Gastroresistant microgranules obtained from Example 1 | 10000 | 90.3 |
| Sodium Croscarmelose (Ac-Di-Sol®) | 552 | 5.0 |
| Microcrystalline cellulose (AvicelPH 102®) | 384 | 3.5 |
| Magnesium stearate | 138 | 1.2 |

The resulting homogeneous mixture was placed in a tabletting machine such as a Killian with concave mold of 10 mm.

The tablets were then coated using a film coating of talc, hydroxypropyl methylcellulose, EDTA sodium salt, 1,2 -propandiol, iron oxide E172. The tablets were heated at the temperature in the range between 41°C and 43°C through a warm air flux and the film coating was sprayed on the tablets.

The total composition of the tablets containing 400 mg of rifaximin as described in Table 5, at the final weight of 626 mg of microgranules correspond to a tablet total weight of 709 mg.

The pharmaceutical composition wherein the rifaximin is in the form of tablets, can contain different amount of rifaximin from 100 mg to 800 mg adding to the microgranules, which contain the corresponding desired amount of rifaximin, a lower or greater amount of diluents, disintegrating and lubricating agents.

**Table 5**

| Composition | Weight (mg) | % (p/p) |
|---|---|---|
| **Microgranules** | | |
| Rifaximin | 400.0 | 56.4 |
| Colloidal anhydrous silica (Aerosil® 200 Pharma) | 2.0 | 0.3 |
| Methacrylic acid ethyl acrylate copolymer (Kollicoat® MAE100 P) | 148.5 | 20.9 |
| 1,2-propandiol | 22.3 | 3.1 |
| Talc | 39.6 | 5.6 |
| Titanium dioxide | 9.9 | 1.4 |

| **Tablet** | | |
|---|---|---|
| Croscarmelose sodium (AcDiSol®) | 34.5 | 4.9 |
| Microcristalline cellulose (Avicel PH 101®) | 24.0 | 3.4 |
| Magnesium stearate | 8.6 | 1.2 |

| **Film coating** | | |
|---|---|---|
| Hydroxypropyl methylcellulose | 14.1 | 2.0 |
| Titanium dioxide | 4.1 | 0.6 |
| 1,2- propandiol | 0.1 | 0.01 |
| EDTA | 1.4 | 0.2 |
| Iron oxide E172 | 0.4 | 0.06 |

The analysis of the resulting tablets by X-ray diffraction showed that the form of rifaximin remains unchanged.

The disintegration time of the tablets has been obtained measuring the disintegration of 8 tablets into 800 ml of demineralised water at temperature of 37 °C ± 1°C in a disaggregator.

The average value corresponds to 8.5 minutes.

The friability value of the tablets was obtained as described on the European Pharmacopea Ed. 6.6. page 5100. Ten tablets were placed in a plastic cylinder where they are exposed to rolling and repeated shocks. After ten minutes, the tablets were weighed. The value of weight loss was expressed as percentage. The weight loss is 0.1%.

The hardness of the tablets was determined as described in European Pharmacopea Ed. 6.0. page 279. Ten tablets were placed in an apparatus such as an ERWEKA TBH-320D. The average value of the hardness obtained was 198 Newtons.

### Example 3

### Preparation of tablets comprising rifaximin in gastroresistant microgranules -Composition 2-

The mixture to prepare the grastroresistant tablets was obtained by mixing 65.15 g of gastroresistant microgranules of rifaximin prepared as described in Example 1, containing 40 g of rifaximin, and 4.15 g of sodium glycolate starch previously sieved on a 0.8 mm mesh sieve. To the resulting mixture was added 0.7 g of magnesium stearate. The composition is reported in Table 6.

**Table 6**

| Composition | Amount (g) | Composition % (p/p) |
|---|---|---|
| Gastroresistant microgranules of rifaximin prepared as in Example 1 | 65.15 | 93.1% |
| Sodium glycolate starch (Explotab®) | 4.15 | 5.9% |
| Magnesium stearate | 0.7 | 1% |

The resultant mixture is placed in a tabletting machine such as a Killian as described in the example 2.

### Example 4

### Preparation of tablets comprising rifaximin in gastroresistant microgranules -Composition 3-

The mixture for the preparation of gastroresistant tablets was obtained by mixing 297.78 g of microgranules gastroresistant of rifaximin prepared as described in the Example 1 and containing 180.47 g of rifaximin, 11.28 of sodium glycolate starch, 4.51 g of cellulose microcrystalline (Avicel PH 120®) previously sieved on a 0.8 mm mesh sieve. Magnesium stearate (2.26 g) is added to the resulting mixture. The composition is described in Table 7.

**Table 7**

| Composition | Amount (g) | % composition (p/p) |
|---|---|---|
| Gastoresistant microgranules prepared as in Example 1 | 297.78 | 94.3% |
| Sodium glycolate starch (Explotab®) | 11.28 | 3.6% |
| cellulose microcrystalline (Avicel PH 102®) | 4.51 | 1.4% |
| Magnesium stearate | 2.26 | 0.7% |

The resultant mixture was placed in a tabletting apparatus such as a Killian as described in the Example 2.

### Example 5

### Preparation of tablets comprising rifaximin in gastroresistant microgranules -Composition 4-

The mixture for the preparation of gastroresistant tablets was obtained by mixing 279.78 g of gastroresistant microgranules of rifaximin prepared as described in the Example 1, containing 180.47 g of rifaximin, and 18.86 g of sodium glycolate starch previously sieved on a 0.8 mm mesh sieve. Magnesium stearate (1.35 g) was added to the resulting mixture of. The composition is reported in Table 8.

**Table 8**

| Composition | Amount (g) | Composition % (p/p) |
|---|---|---|
| Gastroresistant microgranules prepared as in Example 1 | 279.79 | 94.3% |
| Sodium glycolate starch (Explotab®) | 18.86 | 6.3% |
| Magnesium stearate | 1.35 | 0.45% |

The resultant mixture is placed in a tabletting apparatus such as a Killian as described in the Example 2.

### Example 6

### Preparation of tablets comprising rifaximin in gastroresistant microgranules -Composition 5-

The mixture for the preparation of gastroresistant tablets was obtained by mixing 231.0 g of gastroresistant microgranules of prepared as described in the Example 1, containing 139.3 g of rifaximin, 15.6 of sodium glycolate starch, and 13 g of microcrystalline cellulose (Avicel PH 102®) previously sieved on a 0.8 mm mesh sieve. Magnesium stearate (1.0 g) is added to the resulting mixture of. The composition is reported in Table 9.

**Table 9**

| Component | Amount(g) | % composition(p/p) |
|---|---|---|
| Gastoresistant microgranules of rifaximin obtained from Example 1 | 231.00 | 88.64% |
| Sodium glycolate starch (Explotab®) | 15.60 | 5.99% |
| Microcrystalline cellulose (Avicel PH 102®) | 13.00 | 4.99% |
| Magnesium stearate | 1.00 | 0.38% |

The resultant mixture is tabletted using a tabletting apparatus such as Killian as described in the Example 2.

### Example 7

### Preparation of tablets comprising rifaximin in gastroresistant microgranules -Composition 6-

The mixture for the preparation of gastroresistant tablets was obtained by mixing 207.9 g of microgranules gastroresistant of rifaximin prepared as described in the Example 1, containing 117.3 g of rifaximin, 14.1 g of sodium glycolate starch, a mixture of microcrystalline cellulose, composed of 11.7 g of Avicel PH 102® and 12.3 g of Avicel PH101® previously sieved on a 0.8 mm mesh sieve and 1.0 g of magnesium stearate is added to the resulting mixture. The composition is reported in Table 10.

**Table 10**

| Composition | Amount (g) | % composition (p/p) |
|---|---|---|
| Gastroresistant microgranules of rifaximin prepared as in Example 1 | 207.90 | 84.20% |
| Sodium glycolate starch (Explotab®) | 14.10 | 5.71% |
| Microcrystalline cellulose (Avicel PH 102®) | 11.70 | 4.74% |
| Microcrystalline cellulose (Avicel PH 101®) | 12.30 | 4.98% |
| Magnesium stearate | 0.90 | 0.36% |

The resultant mixture was tabletted in a tabletting apparatus such as Killian as described in the Example 2.

### Example 8

### Preparation of tablets comprising rifaximin in gastroresistant microgranules -Composition 7-

The mixture for preparing gastroresistant tablets was obtained mixing 207.9 g of gastroresistant microgranules of rifaximin prepared as described in the Example 1, containing 117.3 g of rifaximin, 14.1 g of sodium glycolate starchand a mixture of microcrystalline cellulose composed of 11.7 g of Avicel PH 102® and 12.3 of Avicel PH 101® previously sieved on a 0.8 mm mesh sieve. 2.15 g of magnesium stearate were added to the resulting mixture. The composition is reported in Table 11.

**Table 11**

| Composition | Amount (g) | % compostion (p/p) |
|---|---|---|
| Gastroresistant microgranules of rifaximin prepared as in Example 1 | 207.90 | 83.78% |
| sodium glycolate starch (Explotab®) | 14.10 | 5.68% |
| Magnesium stearate | 2.15 | 0.87% |
| Microcrystalline cellulose (Avicel PH 102®) | 11.70 | 4.71% |
| Microcrystalline cellulose (Avicel PH 101®) | 12.30 | 4.96% |

The resultant mixture is tabletted using a tabletting apparatus such as Killian as described in the example 2.

### Example 9

### Preparation of tablets comprising rifaximin in gastroresistant microgranules -Composition 8-

The mixture for preparing gastroresistant tablets was obtained mixing 200.9 g of gastroresistant microgranules of rifaximin prepared as described in the Example 1 and containing 113.3 g of rifaximin, 11.3 g of croscarmelose sodium (Ac-Di-Sol®.) and 11.3 g of microcrystalline cellulose (Avicel PH 101®) previously sieved on a 0.8 mm mesh sieve. 2.30 g of magnesium stearate are added to the resulting mixture. The composition is reported in Table 12.

**Table 12**

| Composition | Amount (g) | % composition (p/p) |
|---|---|---|
| Gastroreistant microgranules of rifaximin prepared as in Example 1 | 200.0 | 88.93% |
| Croscarmellose sodium (Ac-Di-Sol®) | 11.30 | 5.02% |
| Cellulose microcrystalline (Avicel PH 101) | 11.30 | 5.02% |
| Magnesium stearate | 2.30 | 1.02% |

### Example 10

### Determination of safety of tablets comprising gastroresistant microgranules containing rifaximin in healthy volunteers and in patients affected by Crohn's disease

Safety of the composition was determined by evaluating before and after administration of the drug, the incidence of adverse affects, vital parameters such as blood pressure, heart rate, body temperature, instrumental evaluations such as electrocardiogram and laboratory analysis, such as blood and urine analysis.

Three tablets comprising 400 mg of rifaximin, obtained as in Example 2 for a total quantity of 1200 mg/day of rifaximin were administered on fasted and fed conditions in 18 healthy volunteers (9 males and 9 females) in a cross-over study with an interval of 8 days between administration in fast and fed condition.

Four adverse advents were reported in the study. In all the cases they were headache of moderate-mild grade not related to the drug administration.

No alteration was shown with regard to vital parameters such as blood pressure, heart rate and biochemical blood and urine analysis after the drug administration.

Safety of pharmaceutical composition in tablets as described in Example 2 has been evaluated in patients affected by inflammatory bowel disease, as for example Crohn's disease.

18 patients affected by active moderate Crohn's disease were administered three tablets of rifaximin 400 mg obtained as in Example 2 for a total quantity of 1200 mg/day.

Safety was determined before and after administration of the medicine by measuring the incidence of adverse affects on vital parameters such as blood pressure, heart rate, body temperature, and instrumental evaluations such as electrocardiogram and laboratory analysis such as blood and urine analysis.

Only one adverse event (anaemia) was registered during the study that was judged by investigators as not drug- related.

No alteration was shown with regard to the biochemical analysis of blood and urine after the administration of the drug. In addition, no significant variation of vital parameters was observed.

From all these results the medicine prepared as in Example 2 is well tolerated.

### Example 11

### Treatment of Crohn's disesase patients with tablets comprising rifaximin in gastroresistant microgranules

The composition in the form of tablets comprising gastroresistant microgranules of rifaximin prepared as in Example 2 was administered in a multicentre randomized double-blind trial versus placebo in patients affected by Crohn's disease.
410 patients affected by in mild-to-moderate active Crohn's disease having a CDAI value between 220 and 400 were enrolled in the trial. Eight patients did not receive any treatment.

The primary end point of the clinical trial was represented by the efficacy of rifaximin to induce clinical remission, defined as a CDAI value lower than 150.

The end points of the clinical trial were also to demonstrate the efficacy of rifaximin to induce clinical response, defined as the reduction of CDAI value of at least 100 points in respect to the basal value; and to demonstrate the maintenance of clinical remission induced by rifaximin treatment for a period of 12 weeks after the suspension of the treatment.

Treatment failure was defined as absence of decrease in CDAI value of at least 70 points after one month of treatment; or increase in CDAI values by 100 points during treatment; or a need for surgery or rescue medication or an increasing of the dosage of concomitant therapy. The patients were divided into four groups:
Group A: 101 patients were administered one tablet of 400 mg rifaximin prepared as in Example 2 comprised in gastroresistant microgranules and two placebo tablets twice a day for a total dosage of 800 mg of rifaximin;
Group B: 104 patients were administered two tablets of 400 mg rifaximin prepared as in Example 2 comprised in gastroresistant microgranules and one placebo tablet twice a day for a total dosage of 1600 mg of rifaximin;
Group C: 98 patients were administered three tablets of 400 mg rifaximin prepared as in Example 2 comprised in gastroresistant microgranules twice a day for a total dosage of 2400 mg of rifaximin;
Group D: 99 patients were administered three placebo tablets twice a day.

All the patients were treated for a period of 12 weeks. During the time of clinical trial the patients could continue to receive concomitant therapy such as 5-ASA and/or immunosuppressant (azatioprine, metotrexate, 6-mercaptopurine, etc) only if they were administered at a stable dosage along the three months before the beginning of the clinical trial and for all the period of clinical trial

At the end of the treatment, the patients who obtained a clinical remission with the therapy were followed for a further period of 12 weeks in order to determine the length of remission after the suspension of the treatment.

The safety profile of the administered treatment was evaluated through the analysis of adverse events; blood and urine laboratory analysis and vital signs such as blood pressure, temperature and heart rate. The clinical trial results reported in Table 13 show that the tablets, prepared as in Example 2 and comprising gastroresistant microgranule of rifaximin obtained as in Example 1, at a daily dosage from 800 mg/day to 2400 mg/day are more efficacious than the placebo in terms of clinical remission, clinical response, and treatment failure rate and the percentage of patients who maintained the remission for the 12 weeks following the end of the treatment. Furthermore the median time to remission was 47 days for patients receiving placebo, 35 days for patients administered Rifaximin-EIR 400 mg twice daily, 27 days for those receiving Rifaximin-EIR 800 mg twice daily and 38 days with Rifaximin-EIR 1200 mg twice daily.

**Table 13**

| | Placebo | Rifaximin (Tablets Example 2) 800 mg/die | Rifaximin (Tablets Example 2) 1600 mg/die | Rifaximin (Tablets Example 2) 2400 mg/die | Rifaximin (Tablets Example 2) Pooled doses |
|---|---|---|---|---|---|
| Clinical remission End of treatment (EOT) | 42.6% (43/101) | 53.8% (56/104) | 62.2% * (61/98) | 47.5% (47/99) | 54.5%* (164/301) |
| Clinical response EOT | 55.9% (52/93) | 62.8% (59/94) | 72.0% * (67/93) | 57.5% (50/87) | 64.2% (176/274) |
| Maintenance of clinical remission after 12 weeks from the end of the treatment | 28.6% (28/98) | 38.2% (39/102) | 44.9% * (40/89) | 31.9% (30/94) | 38.2% (109/285) |
| Time to obtain clinical remission | 47 days | 35 days | 27 days | 38 days | 33 days |
| Treatment failure rate | 44.6% (45/101) | 38.5% (40/104) | 25.5%* (25/98) | 38.4% (38/99) | 34.2% (103/301) |

| | | | | | |
|---|---|---|---|---|---|
| (*) p <0.05 | | | | | |

The overall incidence of adverse events reported during the treatment period and follow-up is summarized in Table 14. The incidence of adverse events and adverse reactions caused by the administration of tablets comprising rifaximin in gastroresistant microgranules at a daily dosage from 800 mg/day to 2400 mg/day is comparable to placebo. There were no significant differences between the study groups.

**Table 14**

| | Placebo | Rifaximin (Tablets Example 2) 800 mg /day | Rifaximin (Tablets Example 2) 1600 mg/day | Rifaximin (Tablets Example 2) 2400 mg/day |
|---|---|---|---|---|
| % Patients with adverse events | 45 | 35 | 38 | 45 |
| % Patients with adverse reaction | 13 | 9 | 8 | 18 |

### Example 12

### Clinical remission in patients affected by Crohn's disease characterized by having a C reactive protein value between 5 mg/l and 10 mg/l with high dosage of rifaximin

The percentage of patients affected by Crohn's disease with the characteristics as in Example 11 and with C reactive protein values at baseline between 5 mg/l and 10 mg/l, after administration of tablets comprising rifaximin obtained as in Example 2 was evaluated.

The clinical results reported in Table 15 demonstrate that the dosage of 2400 mg/day of rifaximin is more efficacious in inducing clinical remission.

**Table 15**

| | Placebo | Rifaximin (Tablets Example 2) 800 mg /day | Rifaximin (Tablets Example 2) 1600 mg/day | Rifaximin (Tablets Example 2) 2400 mg/day | Rifaximin (Tablets Example 2) Pooled doses |
|---|---|---|---|---|---|
| Clinical remission EOT in patients with CPR between 5 and 10 mg/l | 43.8% | 54.5% | 66.7% * | 68.8% | 64.3%* |
| Clinical remission EOT in patients with CPR > 5 mg/l | 36.5% | 47.1% | 62.0% | 46.8% | 52.0% |
| Clinical remission in patients with recent diagnosis of CD | 51.0% | 61.2% | 75.6% | 58.7% | 65.0% |
| Clinical remission EOT in patients with CD localized in ileum-colon and colon | 36.8% | 52.2% | 56.3% | 42.4% | 50.5% |

### Example 13

### Evaluation of the accumulation in the plasma after administration in repeated doses

The composition in the form of tablets comprising gastroresistant microgranules of rifaximin, prepared as in Example 2, was administrated with repeated doses to 12 patients of ages between 18 and 75, affected by mild-to-moderate active Crohn's disease, localized the ileum and/or colon, to evaluate the accumulation of rifaximin in the plasma.

Gastroresistant tablets comprising rifaximin prepared as in Example 2 were administrated to patients, at the dosage of 1200 mg on day 1; 1200 mg twice a day (morning and evening), for a total dosage equal to 2400 mg/day, on days 2, 3, 4, 5, 6; and 1200 mg on day 7, for a total dosage of 14400 mg of rifaximin in seven days.

The plasma concentrations of rifaximin were determined:
the first day before and at 1, 2, 4, 8, 12 and 24 hours from the first administration;
the third and fifth day before the first administration;
the seventh day before the first administration and at 1, 2, 4, 8, 12, 24 and 48 hours from the last administration.

The following pharmacokinetic parameters were determined: the maximum plasmatic concentration of rifaximin (Cₘₐₓ), the area under curve of rifaximin plasmatic concentrations versus time from 0 to 24 hours (AUC₀₋₂₄ₕ) and the time to reach the maximum plasmatic concentration (Tₘₐₓ).

The plasmatic rifaximin concentrations at the pre-dose of day 3 and day 5 were comparable to the plasma concentration at the pre-dose of day 7, demonstrating that the steady state of rifaximin concentrations in plasma was achieved by day 3.

The determination of rifaximin was done through HPLC analysis coupled with a mass spectrometer.

The results are reported in the Table 16.

**Table 16**

| | Cₘₐₓ (ng/ml) | AUC ₀₋₂₄ₕ | Tₘₐₓ |
|---|---|---|---|
| Day 1, fasting condition Administration of rifaximin to 12 patients affected by mild to moderate active Crohn's disease Dose:1200mg | 7.8 | 34.6 | 1.5 |
| Day 7, fasting condition | 6.1 | 41.8 | 3.1 |
| Administration of rifaximin to 12 patients affected by active mild to moderate Crohn's disease Dose: repente dose of 1200mg twice a day | | | |

| | | | |
|---|---|---|---|
| Pt 007/007 early discontinued at Day 1 due to AE not related to study drug; Pt 011/010 was excluded from the analysis due to major protocol deviation (rifaximin at pre-dose samples) | | | |

### Example 14

### Evaluation of the efficacy of rifaximin in the form of gastroresistant tablets in patients affected by Crohn's disease in acute - mild to moderate without any concomitant treatment

The efficacy of the rifaximin in the form of gastroresistant tablets prepared as in Example 2, administrated at daily doses of 800, 1600 and 2400 mg/day, was determined in patients affected by moderate active Crohn's disease who did not receive any concomitant treatment, such as immunosuppressive and/or 5-aminosalycilates, in the three months before the treatment and during the period of the treatment with rifaximin.

The patients are divided into four groups wherein:
Group A: have been treated with one 400 mg tablet rifaximin prepared as in Example 2, administrated twice a day for a total dosage equal to 800 mg/day;
Group B: have been treated with two gastroresistant 400 mg tablets rifaximin prepared as in Example 2, administrated twice a day, for a total dosage equal to 1600 mg/day;
Group C: have been treated with three gastroresistant 400 mg tablets rifaximin 400 mg, prepared as in Example 2, administrated twice a day, for a to dosage equal to 2400 mg/day;
Group D: have been treated with placebo alone.

The results are reported in the Table 17.

**Table 17**

| | Placebo | Rifaximin (Tablets Example 2 | Rifaximin (Tablets Example 2) | Rifaximin (Tables Example 2) |
|---|---|---|---|---|
| | | 800 mg /day | 1600 mg /day | 2400 mg/day |
| N | 8/27 | 13/27 | 17/28 | 18/34 |
| % | 29.6 | 48.1 | 60.7 | 52.9 |
| P | - | 0.1628 | 0.0206 | 0.0674 |

### Example 15

### Evaluation of the effect of rifaximin treatment on faecal water genotoxicity and DNA damage through Comet assay

The effect of the rifaximin treatment on faecal water genotoxicity and DNA damage was assessed through Comet assay, single strand breakage **S**ingle **C**ell **G**el **E**lectrophoresis assay (Comet assay) is a sensitive technique for the detection of DNA damage. It involves the encapsulation of cells in a low-melting-point agarose suspension, lysis of the cells in neutral or alkaline conditions, and electrophoresis of the suspended lysed cells. This is followed by visual analysis with staining of DNA and calculating fluorescence to determine the extent of DNA damage.)

Eleven patients with active Crohn's disease with a CDAI comprised between 150 and 400, were treated with 800 mg rifaximin in tablets, prepared as in Example 2, three time a day corresponding to a total rifaximin quantity of 2400 mg/day for 7 days.
Faecal samples have been collected from the patients before and after rifaximin treatment and faecal water extracted.

Rat immortalized fibroblasts were exposed to faecal water samples at concentration of 1% by weight, collected before and after a seven-day treatment with rifaximin.
Briefly, cell suspensions (1.0 × 105 cells/100 µl) in MEM were incubated in eppendorf tubes for 30 min at 37°C in the presence of faecal water samples (1%). Cells were then transferred on ice and viability was assessed before and after the incubation by trypan blue exclusion. Treatment with 100 mM H₂O₂ or PBS were used as positive and negative controls, respectively. Cells were centrifuged at 4°C at 100 g for 5 min, the supernatant discarded and cells resuspended in low-melting agarose (1.0 × 104 cells/100 µl of 0.5% low-melting agarose in PBS) and immediately pipetted onto agarose-coated slides (1.5% in PBS containing 5mM EDTA). Cells were then covered with a layer of agarose (0.5% in PBS) and allowed to solidify briefly. The slides were immersed in ice-cold lysing solution (2.5M NaCl, 100mM EDTA, 10mM Tris, 1% Sarkosyl, 10% dimethyl sulfoxide and 1% Triton X-100 (pH 10.0)) for 60 min at 4°C. They were then placed on an electrophoretic tray with an alkaline buffer (0.3 N NaOH, 1mM EDTA) and allowed to equilibrate for 20 min at room temperature before the electrophoresis performed at 300mA for 20 min in the same buffer. The slides were then washed, stained for 5 min with 2 mg/ml ethydium bromide (EB) and analyzed with a fluorescence microscope Eclipse E600 (Nikon Corporation, Tokyo, Japan). Images were acquired with a camera coupled with a computer and were analyzed using the software Image-Pro Plus 4.1. This was also used to determine the relationship between migration of nuclear genetic material, or 'head' of the comet, and the resulting 'tail'. DNA damage is expressed as the 'tail moment' which takes into consideration both the quantity of DNA present in the tail and the extent of migration of genetic material (length of the tail). Tail moment was defined as: *TM* = *I***L*, where *I* is the fractional amount of DNA in the comet tail and *L* is the full extent of comet tail from the centre of the comet head to the end of the tail. Tail moment value for the negative controls with PBS, was set at 100. All experiments were performed in triplicate and results were expressed as % ± SD compared to negative control. Positive control (100 mM hydrogen peroxide) value was 420 ± 92 and individual data are reported in Table 1.

In seven patients (Patient No. 1, 2, 3, 4, 6, 10) a significant reduction of DNA strand breakage induced by faecal water was observed after treatment with rifaximin. No differences were observed in patient 5 and 11 because no genotoxicity was observed before the treatment (initial value similar to negative control), while in the other two patients (8, 9) rifaximin did not induce any reduction of DNA strand breakage induced by faecal water: values were high at day 0 and at day 7, at the end of the treatment.

**Table 18**

| **Evaluation of DNA strand breaks induced by fecal water samples collected before and after a seven-days treatment with Rifaximin** | | |
|---|---|---|
| **Negative control** 100 | | |
| **Positive control** (100 mM hydrogen peroxide) 420 ± 92 | | |
| Patient | Day 0 | Day 7 |
| 1 | 280 ± 52 | 120 ± 30 |
| 2 | 360 ± 68 | 147 ± 42 |
| 3 | 260 ± 66 | 126 ± 24 |
| 4 | 240 ± 34 | 124 ± 38 |
| 5 | 122 ± 26 | 120 ± 28 |
| 6 | 248 ± 52 | 1544 ± 56 |
| 8 | 268 ± 47 | 300 ± 64 |
| 9 | 306 ± 42 | 3477 ± 54 |
| 10 | 264 ± 46 | 146 ± 44 |
| 11 | 130 ± 56 | 132 ± 26 |

## Claims

1. Pharmaceutical composition in the form of tablets comprising
i) gastroresistant microgranules containing a hydrate or solvate form of rifaximin β alone or in a mixture with other crystalline, hydrate, solvate or amorphous forms of rifaximin, in an amount comprised between 100 and 800 mg,
ii) pharmaceutically acceptable extragranular excipients, and optionally
iii) a film-forming coating
whereby the total amount of extragranular excipients contained in the tablet does not exceed 30% by weight of the tablet.

2. The pharmaceutical composition according to Claim 1, wherein the total amount of extragranular excipients in the tablet is comprised between 5.0% and 20.0% by weight of the tablet.

3. The pharmaceutical composition according to Claim 1 or 2, wherein the extragranular excipients comprise disintegrants, lubricants and, optionally, diluents.

4. The pharmaceutical composition according to Claim 3, wherein the extra-granular excipients comprise one or more:
- disintegrants, selected from sodium carboxymethylcellulose (carmellose sodium), cross-linked sodium carboxymethylcellulose, (croscarmellose sodium), polyvinylpyrrolidone (povidone), cross-linked polyvinylpolypyrrolidone (crospovidone), sodium starch glycolate, sodium carboxymethyl starch, pregelatinized starch, silica;
- lubricants, selected from magnesium or calcium stearate, sodium stearyl fumarate, hydrogenated vegetable oils, mineral oils, polyethylene glycol, sodium lauryl sulfate, glycerides, sodium benzoate and, optionally,
- diluents, selected from cellulose, microcrystalline cellulose, calcium phosphate, starch, kaolin, calcium sulfate dihydrate, calcium carbonate, lactose, sucrose, glucose, sorbitol, mannitol, glucans, xyloglucans.

5. The pharmaceutical composition according to any one of Claims 1 to 4, comprising coloring agents, sweetening agents and/or antioxidants.

6. The pharmaceutical composition according to any one of Claims 1 to 5, wherein the amount of disintegrants varies between 3.0% and 8.0% by weight, the amount of lubricant varies between 0.3% and 2.0% by weight and the amount of diluents varies between 0.0% and 10.0%, each referred to the total weight of the tablet without coating.

7. The pharmaceutical composition according to any one of Claims 1 to 6 for use in the treatment of intestinal infective and/or inflammatory disease.

8. The pharmaceutical composition for use according to Claim 7, wherein the intestinal infective and/or inflammatory disease is selected from diarrheal syndromes, traveler's diarrhea, diarrheas caused by an altered equilibrium of the intestinal bacterial flora, Crohn's disease, enteritis, ulcerative recto-colitis, irritable bowel syndrome, small intestinal bacterial overgrowth, diverticular disease, hyperammonemia, antibacterial prophylaxis pre- and post-surgery, hepatic encephalopathy, stress articulation disease, interstitial colitis, bacterial peritonitis, infections induced by chronic use of proton pump inhibitors.

9. The pharmaceutical composition for use according to Claim 8 for the treatment of Crohn's disease, wherein the treatment for clinical remission comprises the administration of rifaximin amount comprised between 800 mg and 2400 mg daily.

10. The pharmaceutical composition for use according to Claim 9 for the treatment of Crohn's disease, wherein the treatment period for clinical remission comprises a treatment period of twelve weeks.

11. The pharmaceutical composition for use according to any one of Claims 9 or 10 for the treatment of Crohn's disease, wherein the patient is one having reactive protein C values higher than 5 mg/ml.

12. The pharmaceutical composition for use according to any one of Claims 9 or 10 for the treatment of Crohn's disease, wherein the patient is one having reactive protein C values between about 5 mg/l and 10 mg/ml.

13. The pharmaceutical composition for use according to any one of Claims 9 or 10 for the treatment of Crohn's disease, wherein the patient is one having a recent diagnosis of the disease.

14. The pharmaceutical composition for use according to any one of Claims 9 or 10 for the treatment of Crohn's disease, wherein the patient is one having the disease localized in ileum and colon.

15. The pharmaceutical composition for use according to any one of Claims 9 to 14 as monotherapy.

## Patentansprüche

1. Pharmazeutische Zusammensetzung in Form von Tabletten, umfassend
i) gastroresistente Mikrokügelchen, enthaltend eine Hydrat- oder Solvatform von Rifaximin-β allein oder im Gemisch mit anderen kristallinen, Hydrat-, Solvat- oder amorphen Formen von Rifaximin in einer Menge zwischen 100 und 800 mg,
ii) pharmazeutisch verträgliche extragranuläre Exzipienzien und gegebenenfalls
iii)einen filmbildenden Überzug, wobei die Gesamtmenge der extragranulären Exzipienzien, die in der Tablette enthalten sind, 30 Gewichtsprozent der Tablette nicht überschreitet.

2. Pharmazeutische Zusammensetzung nach Anspruch 1, wobei die Gesamtmenge der extragranulären Exzipienzien in der Tablette zwischen 5,0% und 20,0% bezogen auf das Gewicht der Tablette beträgt.

3. Pharmazeutische Zusammensetzung nach Anspruch 1 oder 2, wobei die extragranulären Exzipienzien Trennmittel, Gleitmittel und gegebenenfalls Verdünnungsmittel umfassen.

4. Pharmazeutische Zusammensetzung nach Anspruch 3, wobei die extragranulären Exzipienzien eines oder mehreres umfassen:
- Trennmittel, ausgewählt aus Natrium-Carboxymethylcellulose (Carmellose-Natrium), vernetzte Natrium-Carboxymethylcellulose (Croscarmellose-Natrium), Polyvinylpyrrolidon (Povidone), vernetztes Polyvinylpyrrolidon (Crospovidone), Natriumstärkeglycolat, Natriumcarboxymethylstärke, vorgelatinisierte Stärke, Siliciumdioxid;
- Gleitmittel, ausgewählt aus Magnesium- oder Calciumstearat, Natriumstearylfumarat, hydrierten Pflanzenölen, Mineralöl, Polyethylenglycol, Natriumlaurylsulfat, Glyceriden, Natriumbenzoat und gegebenenfalls
- Verdünnungsmittel, ausgewählt aus Cellulose, mikrokristalliner Cellulose, Calciumphosphat, Stärke, Kaolin, Calciumsulfatdihydrat, Calciumcarbonat, Laktose, Saccharose, Glucose, Sorbit, Mannit, Glucanen, Xyloglucanen.

5. Pharmazeutische Zusammensetzung nach einem der Ansprüche 1 bis 4, umfassend Farbstoffe, Süßstoffe und/oder Antioxidanzien.

6. Pharmazeutische Zusammensetzung nach einem der Ansprüche 1 bis 5, wobei die Menge der Trennmitteln zwischen 3,0 Gewichtsprozent und 8,0 Gewichtsprozent, die Menge des Gleitmittels zwischen 0,3 Gewichtsprozent und 2,0 Gewichtsprozent und die Menge der Verdünnungsmittel zwischen 0,0% und 10,0%, jeweils bezogen auf das Gesamtgewicht der Tablette ohne Überzug, variieren.

7. Pharmazeutische Zusammensetzung nach einem der Ansprüche 1 bis 6 zur Verwendung zur Behandlung von infektiösen und/oder inflammatorischen Erkrankungen des Darms.

8. Pharmazeutische Zusammensetzung zur Verwendung nach Anspruch 7, wobei die infektiöse und/oder entzündliche Erkrankung des Darms ausgewählt ist aus Diarrhoesyndromen; Reisediarrhoe; Diarrhoen, verursacht durch ein verändertes Gleichgewicht der intestinalen Bakterienflora; Morbus Crohn; Enteritis; ulcerative Rectocolitis; Reizdarmsyndrom; bakterieller Überwucherung des Dünndarms; Divertikelerkrankung; Hyperammonämie; antibakterielle Prophylaxe vor und nach einem chirurgischen Eingriff; hepatischer Enzephalopathie; stressbedingter Gelenkserkrankung; interstitieller Colitis; bakterieller Peritonitis; Infektionen, ausgelöst durch die chronische Verwendung von Protonenpumpenhemmern.

9. Pharmazeutische Zusammensetzung zur Verwendung nach Anspruch 8 zur Behandlung von Morbus Crohn, wobei die Behandlung für klinische Remission die Verabreichung einer Menge an Rifaximin zwischen 800 mg und 2400 mg täglich umfasst.

10. Pharmazeutische Zusammensetzung zur Verwendung nach Anspruch 9 zur Behandlung von Morbus Crohn, wobei die Behandlungsperiode für klinische Remission eine Behandlungsperiode von zwölf Wochen umfasst.

11. Pharmazeutische Zusammensetzung zur Verwendung nach einem der Ansprüche 9 oder 10 zur Behandlung von Morbus Crohn, wobei der Patient einer ist, der Werte an C-reaktivem Protein von größer als 5 mg/ml hat.

12. Pharmazeutische Zusammensetzung zur Verwendung nach einem der Ansprüche 9 oder 10 zur Behandlung von Morbus Crohn, wobei der Patient einer ist, der Werte an C-reaktivem Protein zwischen etwa 5 mg/ml und 10 mg/ml hat.

13. Pharmazeutische Zusammensetzung zur Verwendung nach einem der Ansprüche 9 oder 10 zur Behandlung von Morbus Crohn, wobei der Patient einer ist, bei dem die Erkrankung frisch diagnostiziert wurde.

14. Pharmazeutische Zusammensetzung zur Verwendung nach einem der Ansprüche 9 oder 10 zur Behandlung von Morbus Crohn, wobei der Patient einer ist, bei dem die Krankheit in Ileum und Colon lokalisiert ist.

15. Pharmazeutische Zusammensetzung zur Verwendung nach einem der Ansprüche 9 bis 14 als Monotherapie.

## Revendications

1. Composition pharmaceutique sous forme de cachets comprenant
i) des microgranules gastrorésistants contenant une forme hydratée ou solvatée de rifaximine β seule ou dans un mélange avec d'autres formes cristalline, hydratée, solvatée ou amorphe de rifaximine, dans une quantité comprise entre 100 et 800 mg,
ii) des excipients extragranulaires pharmaceutiquement acceptables, et facultativement
iii) un enrobage filmogène
moyennant quoi la quantité totale d'excipients extragranulaires contenus dans le cachet ne dépasse pas 30 % en poids du cachet.

2. Composition pharmaceutique selon la revendication 1, dans laquelle la quantité totale d'excipients extragranulaires dans le cachet est comprise entre 5,0 % et 20,0 % en poids du cachet.

3. Composition pharmaceutique selon la revendication 1 ou 2, dans laquelle les excipients extragranulaires comprennent des délitants, des lubrifiants et, facultativement, des diluants.

4. Composition pharmaceutique selon la revendication 3, dans laquelle les excipients extragranulaires comprennent un ou plusieurs :
- délitants, choisis parmi la carboxyméthylcellulose sodique (carmellose sodique), la carboxyméthylcellulose sodique réticulée (croscarmellose sodique), la polyvinylpyrrolidone (povidone), la polyvinylpolypyrrolidone réticulée (crospovidone), le glycolate d'amidon sodique, le carboxyméthyl amidon de sodium, l'amidon prégélatinisé, la silice ;
- lubrifiants, choisis parmi le stéarate de magnésium ou de calcium, le stéaryl fumarate de sodium, des huiles végétales hydrogénées, des huiles minérales, le polyéthylène glycol, le lauryl sulfate de sodium, les glycérides, le benzoate de sodium et, facultativement,
- diluants, choisis parmi la cellulose, la cellulose microcristalline, le phosphate de calcium, l'amidon, le kaolin, le sulfate de calcium dihydrate, le carbonate de calcium, le lactose, le saccharose, le glucose, le sorbitol, le mannitol, des glucanes, des xyloglucanes.

5. Composition pharmaceutique selon l'une quelconque des revendications 1 à 4, comprenant des agents colorants, des agents édulcorants et/ou des antioxydants.

6. Composition pharmaceutique selon l'une quelconque des revendications 1 à 5, dans laquelle la quantité de délitants varie entre 3,0 % et 8,0 % en poids, la quantité de lubrifiants varie entre 0,3 % et 2,0 % en poids et la quantité de diluants varie entre 0,0 % et 10,0 %, chacun en référence au poids total du cachet sans enrobage.

7. Composition pharmaceutique selon l'une quelconque des revendications 1 à 6 pour son utilisation dans le traitement d'une maladie intestinale infectieuse et/ou inflammatoire.

8. Composition pharmaceutique pour son utilisation selon la revendication 7, dans laquelle la maladie intestinale infectieuse et/ou inflammatoire est choisie parmi des syndromes de diarrhée, la diarrhée du voyageur, des diarrhées provoquées par un équilibre modifié de la flore bactérienne intestinale, la maladie de Crohn, l'entérite, la recto-colite hémorragique, le syndrome du côlon irritable, une prolifération de bactéries dans le petit intestin, la diverticulose, l'hyperammoiémie, une prophylaxie antibactérienne pré et post-chirurgie, l'encéphalopathie hépatique, une maladie des articulations due au stress, la colite interstitielle, la péritonite bactérienne, des infections induites par une utilisation chronique d'inhibiteurs de la pompe à proton.

9. Composition pharmaceutique pour son utilisation selon la revendication 8 pour le traitement de la maladie de Crohn, dans laquelle le traitement en vue d'une rémission clinique comprend l'administration d'une quantité de rifaximine comprise entre 800 mg et 2 400 mg quotidiennement.

10. Composition pharmaceutique pour son utilisation selon la revendication 9 pour le traitement de la maladie de Crohn, dans laquelle la période de traitement en vue d'une rémission clinique comprend une période de traitement de douze semaines.

11. Composition pharmaceutique pour son utilisation selon l'une quelconque des revendications 9 ou 10 pour le traitement de la maladie de Crohn, dans laquelle le patient est quelqu'un ayant des valeurs de protéine C réactive supérieures à 5 mg/mL.

12. Composition pharmaceutique pour son utilisation selon l'une quelconque des revendications 9 ou 10 pour le traitement de la maladie de Crohn, dans laquelle le patient est quelqu'un ayant des valeurs de protéine C réactive entre environ 5 mg/L et 10 mg/mL.

13. Composition pharmaceutique pour son utilisation selon l'une quelconque des revendications 9 ou 10 pour le traitement de la maladie de Crohn, dans laquelle le patient est quelqu'un à qui on a récemment diagnostiqué la maladie.

14. Composition pharmaceutique pour son utilisation selon l'une quelconque des revendications 9 ou 10 pour le traitement de la maladie de Crohn, dans laquelle le patient est quelqu'un ayant la maladie localisée dans l'iléum et le côlon.

15. Composition pharmaceutique pour son utilisation selon l'une quelconque des revendications 9 à 14 en tant que monothérapie.
